(19) Europäisches Patentamt
European Patent Office
Office européen des brevets

(11) EP 2 496 549 B1

(12) # EUROPÄISCHE PATENTSCHRIFT

(45) Veröffentlichungstag und Bekanntmachung des Hinweises auf die Patenterteilung:
**08.10.2014 Patentblatt 2014/41**

(21) Anmeldenummer: **10773312.3**

(22) Anmeldetag: **02.11.2010**

(51) Int Cl.:
***C07C 271/30*** *(2006.01)* ***C07C 323/43*** *(2006.01)*
***G03F 7/027*** *(2006.01)* ***G03F 7/029*** *(2006.01)*
***G03F 7/031*** *(2006.01)* ***G03F 7/035*** *(2006.01)*
***G03F 7/00*** *(2006.01)*

(86) Internationale Anmeldenummer:
**PCT/EP2010/066633**

(87) Internationale Veröffentlichungsnummer:
**WO 2011/054818 (12.05.2011 Gazette 2011/19)**

(54) **NEUE, NICHT KRISTALLISIERENDE METHACRYLATE, DEREN HERSTELLUNG UND VERWENDUNG**

NEW, NON-CRYSTALLISING METHACRYLATE, ITS MANUFACTURE AND APPLICATION

MÉTHACRYLATES NOUVEAUX NON CRISTALLISANTS, LEUR FABRICATION ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2009 EP 09013763**

(43) Veröffentlichungstag der Anmeldung:
**12.09.2012 Patentblatt 2012/37**

(73) Patentinhaber: **Bayer Intellectual Property GmbH
40789 Monheim (DE)**

(72) Erfinder:
- **FÄCKE, Thomas
51375 Leverkusen (DE)**
- **BRUDER, Friedrich-Karl
47802 Krefeld (DE)**
- **WEISER, Marc-Stephan
51379 Leverkusen (DE)**
- **RÖLLE, Thomas
51381 Leverkusen (DE)**
- **HÖNEL, Dennis
53909 Zülpich-Wichterich (DE)**

(74) Vertreter: **BIP Patents
c/o Bayer Intellectual Property GmbH
Creative Campus Monheim
Alfred-Nobel-Straße 10
40789 Monheim (DE)**

(56) Entgegenhaltungen:
**WO-A1-2008/125199 JP-A- 57 038 750
US-A- 4 420 306**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 496 549 B1

Printed by Jouve, 75001 PARIS (FR)

## Beschreibung

Die Erfindung betrifft ein neues, nicht kristallisierendes Methacrylat sowie ein Verfahren zu dessen Herstellung. Weitere Gegenstände der Erfindung sind eine Photopolymer-Formulierung, umfassend das erfindungsgemäße Methacrylat und die Verwendung der Photopolymerformulierung zur Herstellung holographischer Medien.

Photopolymere stellen Materialien dar, die mittels der Überlagerung zweier kohärenter Lichtquellen belichtet werden können, wobei sich eine dreidimensionale Struktur in den Photopolymeren ausbildet, die sich im allgemeinen durch eine regionale Änderung des Brechungsindexes in dem Material beschreiben lässt. Derartige Strukturen werden Hologramme genannt, die auch als diffraktive optische Elemente beschrieben werden können. Dabei hängt es von der speziellen Belichtung ab, welche optischen Funktionen ein solches Hologramm ausbildet.

In der WO 2008/125199 A1 ist eine Photopolymer-Formulierung beschrieben, die Polyurethanbasierte Matrixpolymere, ein Schreibmonomer auf Acrylatbasis sowie Photoinitiatoren enthält. Im ausgehärtetem Zustand sind in der Polyurethanmatrix das Schreibmonomer und die Photoinitiatoren räumlich isotrop verteilt eingebettet.

Die in der PCT-Anmeldung beschriebenen Acrylat-Schreibmonomere sind in der Herstellung aufwendig, da sie zwingend einen abschließenden Destillationsschritt zur Entfernung des Lösungsmittels benötigen. Dieser ist auch deshalb problematisch, da es dabei zur Polymerisierung der Acrylate kommen kann.

Aufgabe war es nun ein Methacrylat bereit zu stellen, das leicht zugänglich ist, keine Kristallisationsneigung hat und gut in Polyurethannetzwerken löslich ist. Zudem sollte dieses gut polymerisieren können und dabei in der Lage sein, in entsprechenden Photopolymer-Formulierungen Hologramme einzuschreiben zu lassen. Vor allem sollten keine aufwändigen Aufarbeitungsprozeduren bei seiner Herstellung nötig sein.

Diese Aufgabe ist durch ein Methacrylat der allgemeinen Formeln (I) oder (II) sowie Mischungen daraus gelöst

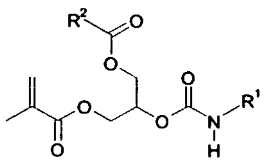

(I)

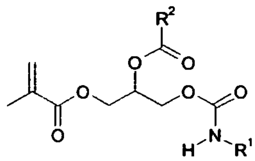

(II)

wobei $R^1$ und $R^2$ unabhängig voneinander substituierte Phenyl-, substituierte und / oder unsubstituierte Naphthylreste sind.

Vorzugsweise können $R^1$ und / oder $R^2$ 6-24 C-Atome, 0-5 S-Atome und 0-5 Halogenatome umfassen.

Gemäß einer bevorzugten Ausführungsform können $R^1$ und /oder $R^2$ mit Thioethergruppen, Phenylgruppen und / oder Halogenatome substituiert sein.

Ganz besonders bevorzugt ist, wenn $R^1$ und / oder $R^2$ Naphthyl, 3-Methylthiophenyl, 2-, 3-, 4-Biphenyl, 2-Bromphenyl sind.

Ein weiterer Gegenstand der Erfindung ist ein Verfahren zur Herstellung eines erfindungsgemäßen Methacrylats, bei dem eine aromatische Säure $R^2$-COOH mit Glycidylmethacrylat zur Reaktion gebracht und das Produkt anschließend mit einem aromatischen Isocyanat $R^1$-NCO umgesetzt wird.

Die Herstellung der erfindungsgemäßen Methacrylate erfolgt in einer 2-Stufen Synthese. In der ersten Umsetzung wird eine Säure $R^2$-COOH mit Glycidylmethacrylat zur Reaktion gebracht, wobei ein Gemisch zweier Alkohole nach Reaktionsschema 1 gebildet wird.

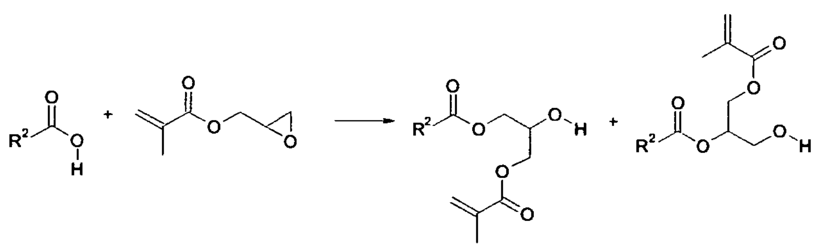

Reaktionsschema 1

Die Umsetzung erfolgt typischerweise bei 20-180 °C, bevorzugt bei 40-120 °C und besonders bevorzugt bei 50-100 °C. Glycidylmethacrylat und ein Katalysator werden vorgelegt und die Säure wird portionsweise zugesetzt. Bedingt durch die begrenzte Löslichkeit wird die Säurezugabe bestimmt durch die Rührbarkeit des Ansatzes. Ein Fortschreiten der Reaktion wird durch das Auflösen der Säure angezeigt. Der Verlauf der Reaktion wird anhand des sich verändernden Epoxidgehalts kontrolliert. $^1$H-NMR Spektroskopie eignet sich hierbei besonders als Nachweisverfahren.

Die Reaktionszeit kann zwischen einigen Stunden bis zu Tagen betragen. Katalysatoren beschleunigen die Umsetzung effizient. Dabei können verschiedene Substanzklassen als Katalysatoren verwendet werden: Beispielsweise. Broenstedsäuren wie Phosphorsäure, phosphorige Säure, Schwefelsäure; Lewissäuren wie Zincacetate, Zinccetylacetonate, Titan-IV-methoxid, Tetrakis(dimethylamino)zirkonium, Lewisbasen wie 2-Methylimidazole, Dimethylaminopyridin, Boran-Pyridin-Komplex, Tris(dimethylamino)boran, Triphenylphosphin, Tris(o-tolyl)phosphin, Choline chloride, Tris(4-dimethylenaminophenyl)phosphin, Tris(4-methoxyphenyl)phosphin, 1,4,5,6-Tetrahydropyrimidin, Diazabicycloundecan (DABCO) und andere Amine, und Ammonium oder Phosphoniumsalze, wie z.B. Tetraethylammoniumtrifluoracetat, Tetrabutylphosphoniumbromid, Benzyltrimethylammonium bromid, Benzyltrimethylammonium chlorid, Tetrabutylphosphoniumchlorid und auch Tetrakis(dimethylamino)silan. Typischerweise werden dabei zwischen 0,01-1%, bevorzugt 0,05-0,2 Gewichts-% des Katalysators verwendet. Triphenylphosphin wird bevorzugt eingesetzt.

In einem zweiten Reaktionsschritt wird das Alkoholgemisch mit einem Monoisocyanat R1-NCO zu dem Methacrylatgemisch gemäß dem Reaktionsschema 2 urethanisiert.

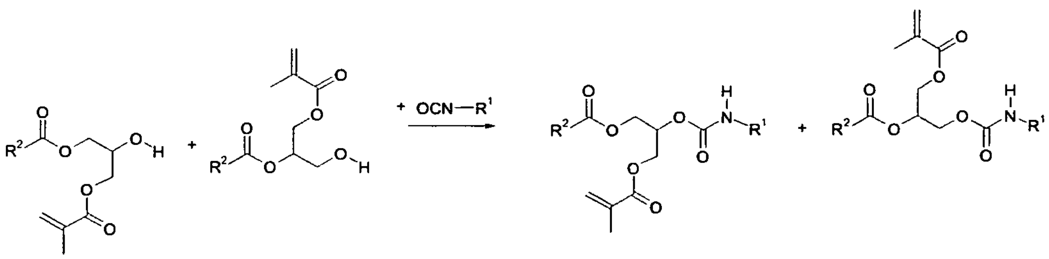


Reaktionschema 2

Die Urethanisierung erfolgt typischerweise bei 20-180 °C, bevorzugt bei 40-120 °C und besonders bevorzugt bei 50-100 °C. Dabei wird der Alkohol als Produkt der ersten Stufen vorgelegt, ggf. mit einem Katalysator versetzt und dann das Isocyanat zugetropft.

Die Reaktion ist beendet, wenn der NCO-Gehalt unter 1%, bevorzugt unter 0,1 Gewichts-% gefallen ist. Der NCO-Gehalt kann dabei mittels IR-Spektroskopie oder durch Titration bestimmt werden.

Es ist möglich das Isomerengemisch mit dem Fachmann bekannten gängigen Verfahren zu trennen. Dabei eignet sich die präparative Säulenchromatografie. Die Trennung kann nach der ersten Stufe oder nach der zweiten Stufe erfolgen.

Es ist ebenfalls möglich zunächst das Isocyanat vorzulegen und den Alkohol anschließend zuzutropfen. Die bevorzugte Art der Zugabe wird im konkreten Fall von der Handhabung und damit von der Viskosität der Ausgangstoffe beeinflusst.

Als Katalysatoren können für die Umsetzung des Reaktionsschemas 2 Amine sowie Metallverbindungen der Metalle Zinn, Zink, Eisen, Bismuth, Molybdän, Kobalt, Calzium, Magnesium und Zirkonium verwendet werden. Bevorzugt sind Zinnoctoat, Zinkoktoat, Dibutylzinndilaurat, Dimethylzinndicarboxylat, Eisen(III)-acetylacetonat, Eisen(II)-chlorid, Zinkchlorid, Tetra-alkyl-ammoniumhydroxide, Alkalihydroxide, Alkalialkoholate, Alkalisalze von langkettigen Fettsäuren mit 10 bis 20 Kohlenstoffatomen und gegebenenfalls seitenständigen OH-Gruppen, Bleioctoat oder tertiäre Amine wie Triethylamin, Tributylamin, Dimethylbenzylamin, Dicyclohexylmethylamin, Dimethyl-cyclohexylamin, N,N,N',N'-Tetramethyl-diamino-diethylether, Bis(dimethylamino-propyl)-harnstoff, N-Methyl- bzw. N-Ethylmorpholin, N,N'-Dimorpholinodiethylether (DMDEE), N-Cyclohexylmorpholin, N,N,N',N'-Tetramethylethylendiamin, N,N,N',N'-Tetramethyl-butandiamin, N,N,N',N'-Tetramethylhexandiamin-1,6, Pentamethyldiethylentriamin, Dimethylpiperazin, N-Dimethylaminoethylpiperidin, 1,2-Dimethylimidazol, N-Hydroxypropylimidazol, 1-Azabicyclo-(2,2,0)-octan, 1,4-Diazabicyclo-(2,2,2)-octan (Dabco) oder Alkanolaminverbindungen, wie Triethanolamin, Triisopropanolamin, N-Methyl- und N-Ethyldiethanolamin, Dimethyl-amino-ethanol, 2-(N,N-Dimethylaminoethoxy)ethanol oder N-Tris-(dialkylaminoalkyl)hexa-hydro-triazine, z.B. N,N',N-Tris-(dimethylaminopropyl)-s-hexahydrotriazin, Diazabicyclononan, Diazabicycloundecan, 1,1,3,3-Tetramethylguanidin, 1,3,4,6,7,8-Hexahydro-1-methyl-2H-pyrimido(1,2-a)pyrimidin.

Besonders bevorzugte Katalysatoren sind hier Dibutylzinndilaurat, Dimethylzinndicarboxylat, Eisen(III)acetylacetonat, 1,4-Diazabicyclo-[2.2.2]-octan, Diazabicyclononan, Diazabicycloundecan, 1,1,3,3-Tetramethylguanidin, 1,3,4,6,7,8-Hexahydro-1-methyl-2H-pyrimido(1,2-a)pyrimidin.

Während der Synthese wird üblicherweise Luft durchgeleitet, um eine unerwünschte Polymerisation zu vermeiden. Dabei muss darauf geachtet werden, dass ausreichend Phenole wie z.B. p-Methoxyphenol oder Jonol anwesend sind, wobei Mengen zwischen 0,001-0,1 Gewichtsprozent ausreichen. Es ist aber auch möglich andere Radikalstabilisatoren zu verwenden, die weiter unten detaillierter beschrieben werden.

Die Isocyanate R1-NCO umfassen Monoisocyanate, wobei R1 die oben aufgeführten Bedeutungen haben kann. Besonders eignen sich die isomeren Methylthiophenylisocyanat wie 2- Methylthiophenylisocyanat, 3- Methylthiophenylisocyanat, 4- Methylthiophenylisocyanat, Bis-, Tris-, Tetra- und Penta(methylthio)phenylisocyanat, Ethylthiophenylisocyanat, n-Propylthiophenylisocyanat, isoPropylthiophenylisocyanat, Butylthiophenylisocyanat, Phenylthiophenylisocyanat, Bis(phenylthio)phenylisocyanat, Naphthylthiophenylisocyanat, Biphenylisocyanat, wie 2-Biphenylisocyanat, 3-Biphenylisocyanat und 4-Biphenylisocyanat, Triphenylisocyanate, Chlorphenylisocyanat, Dichlorphenylisocyanat wie 3,4-Dichlorphenylisocyanat, Tri-, Tetra- und Pentachlorphenylisocyanat und deren Gemische, Bromphenylisocyanat, Di-, Tri, Tetra- und Pentabromphenylisocyanat und deren Gemische. Ebenfalls sind gemischte Substituenten am Phenylisocyanat möglich, wie z.B. Chlorbromphenylisocyanat, Brom(Methylthio)phenylisocyanat, Methylthio(phenyl)-Phenylisocyanat und Analoga.

Substituierte oder nicht substituierte Naphthylisocyanate sind ebenfalls geeignet wie Naphthylisocyanat, PhenylNaphthylisocyanat, ThiomethylNaphthylisocyanat, ThioethylNaphthylisocyanat, ThiopropylNaphthylisocyanat, BromNaphthylisocyanat, ChlorNaphthylisocyanat, sowie mehrfach substituierte und gemischtsubstituierte Naphthylisocyanate.

Bevorzugt sind die isomeren Biphenylisocyanat, Napthylisocyanat, die isomeren Methylthiophenylisocyanat, Bromphenylisocyanat, 3,4-Dichlorophenylisocyanat.

Besonders bevorzugt sind 2-Biphenylisocyanat, 3-Biphenylisocyanat und 4-Biphenylisocyanat, 3-Methylthiophenylisocyanat und Napthylisocyanat.

Geeignete Säuren R2-COOH sind insbesondere aromatische Säuren, wobei diese eine substituierte Benzoesäure oder eine substituierte oder nicht substituierte Naphthylsäure sein können. In der Formel R2-COOH kann R2 die oben genannten Bedeutungen haben.

Bevorzugt können Phenylbenzoesäuren, wie 2-, 3- und 4-Phenylbenzoesäure, sowie die isomere Bis- und Tris-(phenyl)benzoesäuren, die isomeren Naphthylbenzoesäuren, Chlorbenzoesäure, Dichlorbenzoesäure, Trichlorbenzoesäure, Tetrachlorbenzoesäure, Pentachlorbenzoesäure, die isomeren Brombenzoesäuren, Dibrombenzoesäure, Tribrombenzoesäure, Tetrabrombenzoesäure, Pentabrombenzoesäure, Methylthiophenylbenzoesäure, 2- Methylthiophenylbenzoesäure, 3- Methylthiobenzoesäure, 4- Methylthiobenzoesäure, Bis-, Tris-, Tetra- und Penta(methylthio) benzoesäure, Ethylthiobenzoesäure, n-Propylthiobenzoesäure, iso-Propylthiobenzoesäure, Butylthiobenzoesäure, Phenylthiobenzoesäure, Bis(phenylthio)benzoesäure, Naphthylthiobenzoesäure eingesetzt werden.

Besonders bevorzugt sind 2-, 3- und 4-Phenylbenzoesäure, die isomeren Naphthoesäuren, die isomeren Chlorbenzoesäuren, die isomeren Brombenzoesäuren, die isomeren Methylthiobenzoesäuren.

Ganz besonders bevorzugt sind 2-, 3- und 4-Phenylbenzoesäure, 2- Brombenzoesäure und 1-Napthoesäure.

Ein weiterer Gegenstand der Erfindung ist eine Photopolymer-Formulierung, umfassend Matrixpolymere, Schreibmonomere und Photoinitiatoren, wobei die Schreibmonomere ein erfindungemäßes Methacrylat beinhalten.

Geeignete Matrixpolymere sind amorphe Thermoplaste wie Polyacrylate, Polymethylmethacrylate oder Copolymere von Methylmethacrylat, Methacrylsäure oder andere Alkylacrylate und Alkylmethacrylate sowie Acrylsäure; Polyvinylacetat und seine partiell hydrolysierten Derivate wie Polyvinylalkohole, Gelatine, Celluloseester und Celluloseether wie Celluloseacetobutyrat sowie Polyethylenoxide. Besonders bevorzugt handelt es sich bei den Matrixpolymeren um Polyurethane.

Weiterhin eignen sich auch Matrixpolymere auf Basis eines funktionellen Binders und eines Vernetzers. Hierzu können zwei Komponenten Epoxysysteme und Urethansysteme verwendet werden wobei zwei Komponenten Urethansysteme bevorzugt sind. Für die Anwendung der Urethanvernetzung benötigt man einen Polyisocyanatvernetzer und ein hydroxy- oder aminfunktionellen Binder (Harz).

Als Verbindungen der Polyisocyanatvernetzer eignen sich alle dem Fachmann an sich bekannten aliphatischen, cycloaliphatischen, aromatischen oder araliphatischen Di- und Triisocyanate, wobei es unerheblich ist, ob diese mittels Phosgenierung oder nach phosgenfreien Verfahren erhalten wurden. Daneben können auch die dem Fachmann an sich gut bekannten höhermolekularen Folgeprodukte (Oligo- und Polyisocyanate) monomerer Di- und/oder Triisocyanate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Geeignet sind monomere Di- oder Triisocyanate wie Butylendiisocyanat, Hexamethylendiisocyanat (HDI), Isophorondiisocyanat (IPDI), Trimethyl-hexamethylen-diisocyanat (TMDI), 1,8-Diisocyanato-4-(isocyanatomethyl)oktan, Isocyanatomethyl-1,8-octandiisocyanat (TIN), 2,4- und/- oder 2,6-Toluylen-diisocyanat. Ebenfalls geeignet sind auch die Trimerisate des Hexamethylendiisocyanats mit Isocyanurat und / oder Iminooxadiazintrionstruktur.

Ebenfalls möglich ist der Einsatz von isocyanatfunktionellen Prepolymeren mit Urethan-, Allophanat- oder Biuretstrukturen, wie sie in an sich gut bekannter Art und Weise durch Umsetzung der vorgenannten Di-, Tri- oder Polyisocyanate im Überschuss mit hydroxy- oder aminofunktionellen Verbindungen erhalten werden können. Eventuell nicht umgesetztes Ausgangsisocyanat kann anschließend noch entfernt werden, um monomerenarme Produkte zu erhalten. Zur Beschleunigung der Prepolymerbildung kann der Einsatz von dem Fachmann an sich aus der Polyurethanchemie gut bekannten Katalysatoren hilfreich sein.

Bevorzugt geeignet sind Oligo- und Polyisocyanate abgeleitet von monomeren Diisocyanate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur, die jeweils einzeln oder in beliebigen Mischungen untereinander eingesetzt werden.

Besonders bevorzugt sind Oligo- und Polyisocyanate aliphatischer Diisocyanate mit Isocyanurat-, Allophanat-, Biuret-, Uretdion-, Iminooxadiazindionstruktur, die jeweils einzeln oder in beliebigen Mischungen untereinander eingesetzt werden.

Geeignete hydroxy- oder aminfunktionelle Binder (Harze) sind Di- oder Polyole und/oder -amine mit einem zahlenmittleren Molekulargewicht im Bereich von 500 bis 13000 g/mol, bevorzugt 700 bis 8500 g/mol.

Bevorzugte Harze zu diesem Zweck besitzen eine mittlere Funktionalität von 1.5 bis 3.5, bevorzugt von 1.8 bis 3.2, besonders bevorzugt von 1.9 bis 3.1.

Solche Polyole der vorstehend genannten Art sind beispielsweise Polyesteralkohole auf Basis von aliphatischen, cycloaliphatischen und/oder aromatischen Di-, Tri- und/oder Polycarbonsäuren mit Di-, Tri-, und/oder polyfunktionellen Alkoholen sowie Polyesteralkohole auf Lacton-Basis.

Bevorzugte Polyesteralkohole mit einem Molekulargewicht bevorzugt von 500 bis 4000, besonders bevorzugt 650 bis 2500 g/mol sind z.B. Umsetzungsprodukte von Adipinsäure mit Hexandiol, Butandiol oder Neopentylglykol oder Mischungen der genannten Diole.

Ebenfalls geeignet sind Polyetherpolyole, die durch Polymerisation von cyclischen Ethern oder durch Umsetzung von Alkylenoxiden mit einem Startermolekül erhältlich sind.

Beispielhaft seien genannt die Polyethylen- und/oder Polypropylenglykole eines zahlenmittleren Molekulargewichts von 500 bis 13000 g/mol, weiterhin Polytetrahydrofurane eines zahlenmittleren Molekulargewichts von 500 bis 8000, bevorzugt von 650 bis 3000 g/mol.

Bevorzugte Polyetherpolyole sind Polyethylen/Polypropylenglykole mit einem Polypropylengehalt von mindestens 70% und einer Funktionalität von 1.9 bis 3.1.

Ebenfalls geeignet sind Polyester-Polyether-Polyester-Blockpolyole, die durch Umsetzung von Polyetherpolyolen mit Lactonen erhalten werden können.

Bevorzugt sind Polyester-Polyether-Polyester-Blockpolyole, dabei sind besonders bevorzugt Polyester-Polyether-Polyester-Blockpolyole auf Basis von Polytetrahydrofuranen mit einem zahlenmittleren Molekulargewichts von 200 bis 2000 g/mol und ε-Caprolacton, wobei diese Polyester-Polyether-Polyester-Blockpolyole ein zahlenmittleres Molekulargewicht von 1000 bis 8000 g/mol haben.

Ebenfalls geeignet sind hydroxyl-terminierte Polycarbonate, die durch Umsetzung von Diolen oder auch Lactonmodifizierten Diolen oder auch Bisphenolen, wie z.B. Bisphenol A, mit Phosgen oder Kohlensäurediestern wie Diphenylcarbonat oder Dimethylcarbonat zugänglich sind.

Beispielhaft seien genannt die polymeren Carbonate des 1,6-Hexandiols eines zahlenmittleren Molekulargewichts von 500 bis 8000 g/mol, sowie die Carbonate von Umsetzungsprodukten des 1,6-Hexandiols mit ε-Caprolacton im molaren Verhältnis von 1 bis 0.1. Bevorzugte Carbonate sind vorgenannte Polycarbonatdiole eines zahlenmittleren Molekulargewichts von 650 bis 3000 g/mol auf Basis 1,6-Hexandiol und/oder Carbonate von Umsetzungsprodukten des 1,6-Hexandiols mit ε-Caprolacton im molaren Verhältnis von 1 bis 0.33.

Hydroxyl-terminierte Polyamidalkohole und Hydroxyl-terminierte Polyacrylatdiole, z.B. Tegomer® BD 1000 (Fa. Tego GmbH, Essen, Deutschland) sind ebenfalls einsetzbar.

Besonders bevorzugt sind Polyethylen/Polypropylenglykole mit einem Polypropylengehalt von mindestens 70% und einer Funktionalität von 1.9 bis 2.5 sowie Polyester-Polyether-Polyester-Blockpolyole auf Basis von Polytetrahydrofuranen mit einem zahlenmittleren Molekulargewichts von 400 bis 1400 g/mol und ε-caprolacton, wobei diese Polyester-Polyether-Polyester-Blockpolyole ein zahlenmittleres Molekulargewichts von 1500 bis 4000 g/mol haben.

Photoinitiatoren sind üblicherweise durch aktinische Strahlung aktivierbare Initiatoren, die eine Polymerisation der entsprechenden polymerisierbaren Gruppen auslösen. Photoinitiatoren sind an sich bekannte, kommerziell vertriebene Verbindungen, wobei zwischen unimolekularen (Typ I) und bimolekularen (Typ II) Initiatoren unterschieden wird. Desweiteren werden diese Initiatoren je nach chemischer Natur für die radikalische, die anionische (oder), die kationische (oder gemischte) Formen der vorgenannten Polymerisationen eingesetzt. Die Photoinitiatoren können vorzugsweise einen anionischen, kationischen oder neutralen Farbstoff und einen Coinitiator umfassen.

(Typ I)-Systeme für die radikalische Photopolymerisation sind z.B. aromatische Ketonverbindungen, z.B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacyclophosphinoxide, Phenylglyoxylsäureester, Campherchinon, alpha-Aminoalkylphenone, alpha-,alpha-Dialkoxyacetophenone, 1-[4-(Phenylthio)phenyl]octan-1,2-dion-2-(O-benzoyloxim), unterschiedlich substituierte Hexarylbisimidazole (HABI) mit geeigneten Coinitiatoren wie z.B. Mer-captobenzoxazol sowie alpha-Hydroxyalkylphenone. Auch die in EP-A 0223587 beschriebenen Photoinitiatorsysteme bestehend aus einer Mischung aus

einem Ammoniumarylborat und einem oder mehreren Farbstoffen können als Photoinitiator eingesetzt werden. Als Ammoniumarylborat eignen sich beispielsweise Tetrabutylammonium Triphenylhexylborat, Tetrabutylammonium Triphenylbu-tylborat, Tetrabutylammonium Trinapthylbutylborat, Tetramethylammonium Triphenylbenzylborat, Tetra(n-hexyl)ammonium (sec-Butyl)triphenylborat, 1-Methyl-3-octylimidazolium Dipentyldiphenylborat, Tetrabutylammonium Tris-(4-tert.-butyl)-phenylbutylborat, Tetrabutylammonium Tris-(3-fluorphenyl)-hexylborat und Tetrabutylammonium Tris-(3-Chlor-4-methylphenyl)-hexylborat. Als Farbstoffe eignen sich beispielsweise Neu-Methylenblau, Thionin, Basic Yellow, Pinacynol Chlorid, Rhodamin 6G, Gallocyanin, Ethylviolett, Victoria Blue R, Celestine Blue, Chinaldinrot, Kristallviolett, Brilliant Grün, Astrazon Orange G, Darrow Red, Pyronin Y, Basic Red 29, Pyrillium I, Safranin O, Cyanin und Methylenblau, Azur A (Cunningham et al., RadTech'98 North America UV/EB Conference Proceedings, Chicago, Apr. 19-22, 1998).

Die für die anionische Polymerisation verwendeten Photoinitiatoren sind in der Regel (Typ I)-Systeme und leiten sich von Übergangsmetall-Komplexen der ersten Reihe ab. Hier sind ChromSalze, wie z.B. trans-$Cr(NH_3)_2(NCS)_4$-(Kutal et al, Macromolecules 1991, 24, 6872) oder Ferrocenyl-Verbindungen (Yamaguchi et al. Macromolecules 2000, 33, 1152). Eine weitere Möglichkeit der anionischen Polymerisation besteht in der Verwendung von Farbstoffen, wie Kristallviolett Leukonitril oder Malachit Grün Leukonitril, die durch photolytischen Zerfall Cyanoacrylate polymerisieren können (Neckers et al. Macromolecules 2000, 33, 7761). Allerdings wird dabei das Chromophor in das Polymer eingebaut, so dass die resultierenden Polymere durchgefärbt sind.

Die für die kationische Polymerisation verwendeten Photoinitiatoren bestehen im wesentlichen aus drei Klassen: Aryldiazonium-Salze, Onium-Salze (hier speziell: Iodonium-, Sulfonium- und Selenonium-Salze) sowie Organometall-Verbindungen. Phenyldiazonium-Salze können unter Bestrahlung sowohl in Gegenwart als auch in Abwesenheit eines Wasserstoff-Donors ein Kation erzeugen, das die Polymerisation initiiert. Die Effizienz des Gesamtsystems wird durch die Natur des verwendeten Gegenions zur Diazonium-Verbindung bestimmt. Bevorzugt sind hier die wenig reaktiven aber recht teuren $SbF_6^-$, $AsF_6^-$ oder $PF_6^-$. Für den Einsatz in Beschichtung dünner Filme sind diese Verbindungen i.d.R wenig geeignet, da die den nach der Belichtung freigesetzten Stickstoff die Oberflächegüte herabgesetzt wird (pinholes) (Li et al., Polymeric Materials Science and Engineering, 2001, 84, 139). Sehr weit verbreitet und auch in vielerlei Form kommerziell erhältlich sind Onium-Salze, speziell Sulfonium- und Iodonium-Salze. Die Photochemie dieser Verbindungen ist nachhaltig untersucht worden. Die Iodonium-Salze zerfallen nach der Anregung zunächst homolytisch und erzeugen somit ein Radikal und ein Radikalanion, welches sich durch H-Abstraktion stabilisiert und ein Proton freisetzt und dann die kationische Polymerisation startet (Dektar et al. J. Org. Chem. 1990, 55, 639; J. Org. Chem., 1991, 56. 1838). Dieser Mechanimus ermöglicht den Einsatz von Iodonium-Salzen ebenfalls für die radikalische Photopolymerisation. Hierbei kommt erneut der Wahl des Gegenions eine große Bedeutung zu, bevorzugt werden ebenfalls $SbF_6^-$, $AsF_6^-$ oder $PF_6^-$. Ansonsten in dieser Strukturklasse die Wahl der Substitution des Aromaten recht frei und im wesentlichen durch die Verfügbarkeit geeigneter Startbausteine für die Synthese bestimmt. Bei den Sulfonium-Salzen handelt es sich um Verbindungen, die nach Norrish(II) zerfallen (Crivello et al., Macromolecules, 2000, 33, 825). Auch bei den Sulfonium-Salzen kommt der Wahl des Gegenions eine kritische Bedeutung zu, die sich im Wesentlichen in der Härtungsgeschwindigkeit der Polymere äußert. Die besten Ergebnisse werden i.d.R. mit $SbF_6^-$ Salzen erzielt. Da die Eigenabsorption von Iodonium- und Sulfonium-Salze bei <300nm liegt, müssen diese Verbindungen für die Photopolymerisation mit nahem UV oder kurzwelligem sichtbarem Licht entsprechend sensibilisiert werden. Dies gelingt durch die Verwendung von höher absorbierenden Aromaten wie z.B. Anthracen und Derivaten (Gu et al., Am. Chem. Soc. Polymer Preprints, 2000, 41 (2), 1266) oder Phenothiazin bzw. dessen Derivate (Hua et al, Macromolecules 2001, 34, 2488-2494).

Es kann vorteilhaft sein auch Gemische dieser Verbindungen einzusetzen. Je nach zur Härtung verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Tech-nology of UV & EB Formulations For Coatings, Inks & Paints, Vol. 3, 1991, SITA Tech-nology, London, S. 61 - 328 beschrieben.

Bevorzugte Photoinitiatoren sind Mischungen aus Tetrabutylammonium Triphenylhexylborat, Tetrabutylammonium Triphenylbutylborat, Tetrabutylammonium Trinapthylbutylborat, Tetrabutylammonium Tris-(4-tert.-butyl)-phenylbutylborat, Tetrabutylammonium Tris-(3-fluorphenyl)-hexylborat und Tetrabutylammonium Tris-(3-Chlor-4-methylphenyl)-hexylborat mit Farbstoffen wie beispielsweise Astrazon Orange G, Methylenblau, Neu Methylenblau, Azur A, Pyrillium I, Safranin O, Cyanin, Gallocyanin, Brilliant Grün, Kristallviolett, Ethylviolett und Thionin.

Ferner können in die erfindungsgemäßen Formulierungen auch Radikalstabilisatoren, Katalysatoren, Weichmacher und weitere Zusatzstoffe mit eingesetzt werden.

Als Radikalstabilisatoren geeignet sind Inhibitoren und Antioxidantien wie sie in "Methoden der organischen Chemie" (Houben-Weyl), 4. Auflage, Band XIV/1, S. 433ff, Georg Thieme Verlag, Stuttgart 1961, beschrieben sind. Geeignete Stoffklassen sind beispielsweise Phenole wie z.B. 2,6Di-tert-butyl-4-methylphenol, Kresole, Hydrochinone, Benzylalkohole wie z.B. Benzhydrol, ggf. auch Chinone wie z. B. 2,5-Di-tert.-Butylchinon, ggf. auch aromatische Amine wie Diisopropylamin oder Phenothiazin. Bevorzugte Radikalstabilisatoren sind 2,6-Di-tert.-butyl-4-methylphenol, Phenothiazin und Benzhydrol.

Ferner können ein oder mehrere Katalysatoren eingesetzt werden. Diese katalysieren bevorzugt die Urethanbildung. Dies sind generell die gleichen Katalysatoren, die auch in der zweiten Reaktionsstufe bei der Herstellung der erfindungsgemäßen Methacrylate verwendet werden (siehe oben).

Als weitere Hilfs- und Zusatzstoffe können beispielsweise enthalten sein Lösemittel, Weichmacher, Verlaufsmittel, Benetzungsmittel, Entschäumer oder Haftvermittler, aber auch Polyurethane, thermoplastische Polymere, Oligomere, weitere funktionelle Gruppen, wie z.B. Acetale, Epoxid, Oxetane, Oxazoline, Dioxolane und/oder hydrophile Gruppen, wie z.B. Salze und/oder Polyethylenoxide, aufweisende Verbindungen.

Als Lösemittel werden dabei bevorzugt leichtflüchtige Lösemittel mit guter Vemäglichkeit mit den erfindungswesentlichen Formulierungen verwendet, beispielsweise Ethylacetat, Butylacetat, Aceton.

Als Weichmacher werden dabei bevorzugt Flüssigkeiten mit guten Löseeigenschaften, geringer Flüchtigkeit und hoher Siedetemperatur eingesetzt. Geeignete Weichmacher sind die in der Polymerchemie bekannten Verbindungen wie Ester aromatischer Säuren wie z.B. Phthalsäuredibutylester, Triisononyltrimellitat oder Diethylenglykoldibenzoat; die Alkylsulfonsäureester des Phenols; Ester aliphatischer Säuren wie z.B. Cyclohexan-1,2-dicarbonsäurediisononylester, Acetyltributylcitrat, Dibutylsebacat, Adipinsäurepolyester oder Dibutyladipat; Essigsäureester wie z.B. Glycerintriacetat, Ester ungesättigter Säuren wie Di(2-ethylhexyl)maleat; Ester der Phosphorsäure wie z.B. Tributoxyethylphosphat; Sulfonamide wie z.B. N-Butyl-Benzensulfonamid; Mineralöle wie aromatische Öle, naphthenische Öle, und paraffinische Öle; Pflanzenöle wie Campher, epoxidiertes Sojaöl oder Leinöl, Rizinusöl und Ether kurzkettiger Alkohole und Ether, wie z.B. Hexandioldibutylether oder Triethylenglycoldünethylether.

Die Photopolymer-Formulierung kann zusätzlich Urethane als Weichmacher enthalten, wobei die Urethane insbesondere mit wenigstens einem Fluoratom substituiert sein können. Bevorzugt können die Urethane die allgemeine Formel (5)

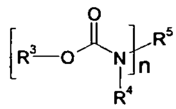
(5)

haben, in der n≥1 und n≤8 ist und $R^3$, $R^4$, $R^5$ Wasserstoff und / oder unabhängig voneinander lineare, verzweigte, cyclische oder heterocyclische unsubstituierte oder gegebenenfalls auch mit Heteroatomen substituierte organische Reste sind, wobei bevorzugt mindestens einer der Reste $R^3$, $R^4$, $R^5$ mit wenigstens einem Fluoratom substituiert ist und besonders bevorzugt $R^3$ ein organischer Rest mit mindestens einem Fluoratom ist.

Es kann auch von Vorteil sein, gleichzeitig mehrere Zusatzstoffe eines Typs zu verwenden. Selbstverständlich kann es ebenfalls von Vorteil sein, mehrere Zusatzstoffe mehrerer Typen zu verwenden.

Bei einer weiteren bevorzugten Ausführungsform ist vorgesehen, dass die Schreibmonomere zusätzlich ein multifunktionales Schreibmonomer umfassen, wobei es sich insbesondere um ein multifunktionelles Acrylat handeln kann. Dabei kann das multifunktionelle Acrylat insbesondere die allgemeine Formel (IV)

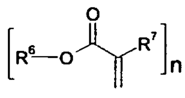
(IV)

aufweisen, in der n≥2 und n≤4 ist und $R^6$, $R^7$ Wasserstoff und / oder unabhängig voneinander lineare, verzweigte, cyclische oder heterocyclische unsubstituierte oder gegebenenfalls auch mit Heteroatomen substituierte organische Reste sind.

Ebenso ist es möglich, dass weitere ungesättigte Verbindungen wie $\alpha,\beta$-ungesättigte Carbonsäurederivate wie Acrylate, Methacrylate, Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, $\alpha$-Methylstyrol, Vinyltoluol, Olefine, wie z.B. 1-Octen und/oder 1-Decen, Vinylestern, (Meth)acrylnitril, (Meth)acrylamid, Methacrylsäure, Acrylsäure zugesetzt werden. Bevorzugt sind Acrylate und Methacrylate.

Als Acrylate bzw. Methacrylate werden allgemein Ester der Acrylsäure bzw. Methacrylsäure bezeichnet. Beispiele verwendbarer Acrylate und Methacrylate sind Methylacrylat, Methylmethacrylat, Ethylacrylat, Ethylmethacrylat, Ethoxyethylacrylat, Ethoxyethylmethacrylat, n-Butylacrylat, n-Butylmethacrylat, tert.-Butylacrylat, tert.-Butylmethacrylat, Hexylacrylat, Hexylmethacrylat, 2Ethylhexylacrylat, 2-Ethylhexylmethacrylat, Butoxyethylacrylat, Butoxyethylmethacrylat, Laurylacrylat, Laurylmethacrylat, Isobornylacrylat, Isobornylmethacrylat, Phenylacrylat, Phenylmethacrylat, p-Chlorphenylacrylat, p-Chlorphenylmethacrylat, p-Bromphenylacrylat, p-Bromphenyl-methacrylat, 2,4,6-Trichlorphenylacrylat, 2,4,6-Trichlorphenylmethacrylat, 2,4,6-Tribromphenyl-acrylat, 2,4,6-Tribromphenylmethacrylat, Pentachlorphenylacrylat, Pentachlorphenylmethacrylat, Pentabromphenylacrylat, Pentabromphenylmethacrylat, Pentabrombenzylacrylat, Pentabrombenzylmethacrylat, Phenoxyethylacrylat, Phenoxyethylmethacrylat, Phenoxyethoxyethylacrylat,

Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2butylmethacrylat, Propan-2,2-diylbis[(2,6-dibrom-4,1-phenylen)oxy(2-{[3,3,3-tris(4-chlorphenyl)propanoyl]-oxy}propan-3,1-diyl)oxyethan-2,1-diyl]-diacrylat, Bisphenol A Diacrylat, Bisphenol A Dimethacrylat, Tetrabromobisphenol A Diacrylat, Tetrabromobisphenol A Dimethacrylat sowie deren ethoxylierte Analogverbindungen, N-Carbazolylacrylate, um nur eine Auswahl verwendbarer Acrylate und Methacrylate zu nennen.

Selbstverständlich können auch weitere Urethanacrylate verwendet werden. Unter Urethanacrylaten versteht man Verbindungen mit mindestens einer Acrylsäureestergruppe, die zusätzlich über mindestens eine Urethanbindung verfügen. Es ist bekannt, dass solche Verbindungen durch Umsetzung eines Hydroxy-funktionellen Acrylsäureesters mit einer Isocyanat-funktionellen Verbindung erhalten werden können.

Beispiele hierfür verwendbarer Isocyanate sind aromatische, araliphatische, aliphatische und cycloaliphatische Di-, Tri- oder Polyisocyanate. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisocyanat (HDI), Isophorondiisocyanat (IPDI), 1,8-Diisocyanato-4-(isocyanatomethyl)oktan, 2,2,4- und/oder 2,4,4-Trimethylhexamethylendiisocyanat, die isomeren Bis(4,4'-isocyanatocyclohexyl)methane und deren Mischungen beliebigen Isomerengehalts, Isocyanatomethyt-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, 1,5-Naphthylendiisocyanat, m-Methylthiophenylisocyanat ,Triphenylmethan-4,4',4"-triisoc y a n a t u n d T r i s ( p-isocyanatophenyl)thiophosphat oder deren Derivate mit Urethan-, Harnstoff-, Carbodiimid-, Acylhamstoff-, Isocyanurat-, Allophanat-, Biuret-, Oxadiazintrion-, Uretdion-, Iminooxadiazindionstruktur und Mischungen derselben. Bevorzugt sind dabei aromatische oder araliphatische Di-, Tri- oder Polyisocyanate.

Als hydroxyfunktionelle Acrylate oder Methacrylate für die Herstellung von Urethanacrylaten kommen beispielsweise Verbindungen in Betracht wie 2-Hydroxyethyl(meth)acrylat, Polyethylenoxidmono(meth)acrylate, Polypropylenoxidmono(meth)acrylate, Polyalkylenoxidmono(meth)-acrylate, Poly(ε-caprolacton)mono(meth)acrylate, wie z.B. Tone® M100 (Dow, Schwalbach, DE), 2Hydroxypropyl(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylate. Darüber hinaus sind isocyanat-reaktive oligomere oder polymere ungesättigte Acrylat- und/oder Methacrylatgruppen enthaltende Verbindungen alleine oder in Kombination mit den vorgenannten monomeren Verbindungen geeignet. Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(pisocyanatophenyl)thiophosphat und m-Methylthiophenylisocyanat mit alkoholfunktionellen Acrylaten wie Hydroxyethyl(meth)acrylat, Hydroxypropyl(meth)acrylat und Hydroxybutyl(meth)acrylat.

Ein weiterer Gegenstand der Erfindung ist die Verwendung einer erfmdungsgemäßen Photopolymer-Formulierung zur Herstellung holographischer Medien, die durch entsprechende Belichtungsprozesse für optische Anwendungen im gesamten sichtbaren und nahen UV-Bereich (300-800 nm) zu Hologrammen verarbeitet werden können. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme"), Denisyukhologramme, Off-Axis Reflektionshologramme, Edge-Lit Hologramme sowie holographische Stereogramme, bevorzugt sind Reflexionshologramme, Denisyukhologramme, Transmissionshologramme. Mögliche optische Funktionen der Hologramme, die mit den erfindungsgemäßen Photopolymerzusammensetzungen hergestellt werden können entsprechen den optische Funktionen von Lichtelementen wie Linsen, Spiegel, Umlenkspiegel, Filter, Streuscheiben, Beugungselemente, Lichtleiter, Lichtlenker (waveguides), Projektionsscheiben und/oder Masken. Häufig zeigen diese optischen Elemente eine Frequenzselektivität je nachdem wie die Hologramme belichtet wurden und welche Dimensionen das Hologramm hat.

Zudem können mittels der erfindungsgemäßen Photopolymerzusammensetzungen auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellen Produkten. Holographische Bilder können den Eindruck eines dreidimensionalen Bildes haben, sie können aber auch Bildsequenzen,

kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme, eine attraktive technische Lösung für die oben genannten Anwendung dar.

Die Photopolymere-Formulierungen können insbesondere als holografisches Medium in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Alternativ zu den vorgenannten Kunststoffträgern können auch planare Glasplatten eingesetzt werden, die insbesondere für großflächige abbildungsgenaue Belichtungen Verwendung finden, z.B. für holographische Lithographie [Ng, Willie W.; Hong, Chi-Shain; Yariv, Amnon. Holographie interference lithography for integrated optics. IEEE Transactions on Electron Devices (1978), ED-25(10), 1193-1200. ISSN:0018-9383].

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rollezu-Rolle-Verfahren, gefordert sind.

## Beispiele

Die Erfindung wird im Folgenden anhand von Beispielen näher erläutert.

### Messung der holographischen Eigenschaften DE und $\Delta n$ der holographischen Medien mittels Zweistrahlinterferenz in Reflexionsanordnung

Die so herstellbaren holografischen Medien wurden anschließend mittels einer Messanordnung gemäß Figur 1 wie folgt auf ihre holographischen Eigenschaften geprüft:

Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel ($\alpha_0$) des Referenzstrahls beträgt -21.8°, der Einfallswinkel ($\beta_0$) des Signalstrahls beträgt 41.8°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand Λ, auch Gitterperiode genannt, im Medium beträgt ~ 225 nm (der Brechungsindex des Mediums zu ~1.504 angenommen).

Figur 1 zeigt den holographischen Versuchsaufbau, mit dem die Beugungseffizienz (DE) der Medien gemessen wurde. Figur 1 zeigt die Geometrie eines Holographie Media Testers (HMT) bei $\lambda$ = 633 nm (He-Ne Laser): M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -21.8°, $\beta_0$ = 41.8° sind die Einfallswinkel der kohärenten Strahlen außerhalb der Probe (des Mediums) gemessen. RD = Referenzrichtung des Drehtisches.

Es wurden auf folgende Weise Hologramme in das Medium geschrieben:

- Beide Shutter (S) sind für die Belichtungszeit t geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls

blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording}.$$

$\theta_0$ ist der Halbwinkel im Laborsystem außerhalb des Mediums und es gilt beim Schreiben des Hologramms:

$$\theta_0 = \frac{\alpha_0 - \beta_0}{2}.$$

In diesem Fall gilt also $\theta_0$ = -31.8°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwüikels $\Omega$ des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$ ermittelt. Eventuell musste dazu die Position des Detektors des abgebeugten Strahls verändert werden, um diesen maximalen Wert zu bestimmen.

Der Brechungsindexkontrast $\Delta n$ und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand Λ' des Hologramms und die Orientierung der Streifen (slant) vom Streifenabstand Λ des Interferenzmusters und dessen Orientierung abweichen kann. Demnach wird auch der Winkel $\alpha_0$' bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve $\eta(\Omega)$ eines Reflexionshologramms gilt nach Kogelnik:

$$\eta = \begin{cases} \dfrac{1}{1 - \dfrac{1-(\xi/\nu)^2}{\sin^2\left(\sqrt{\xi^2 - \nu^2}\right)}}, \text{für } \nu^2 - \xi^2 < 0 \\ \dfrac{1}{1 + \dfrac{1-(\xi/\nu)^2}{\sinh^2\left(\sqrt{\nu^2 - \xi^2}\right)}}, \text{für } \nu^2 - \xi^2 \geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left(2 \cdot \cos(\psi' - \vartheta') - \frac{\lambda}{n \cdot \Lambda'}\right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$

$$\sin(\vartheta'_0) = n \cdot \sin(\vartheta')$$

An der Bragg-Bedingung ist das "Dephasing" *DP*= 0. Und es folgt entsprechend:

$$\alpha'_0 = \theta_0 + \Omega_{reconstruction}$$

$$\sin(\alpha'_0) = n \cdot \sin(\alpha')$$

Der noch unbekannt Winkeln β' kann aus dem Vergleich der Bragg-Bedingung des Interferenzfeldes beim

Schreiben des Hologramms und der Bragg-Bedingung beim Auslesen des Hologramms ermittelt werden unter der Annahme, dass nur Dickenschwindung stattfindet. Dann folgt:

$$\sin(\beta') = \frac{1}{n} \cdot [\sin(\alpha_0) + \sin(\beta_0) - \sin(\theta_0 + \Omega_{reconstruction})]$$

$\nu$ ist die Gitterstärke, $\xi$ ist der Detuning Parameter und $\psi'$ die Orientierung (Slant) des Brechungsindexgitters das geschrieben wurde. $\alpha'$ und $\beta'$ entsprechen den Winkeln $\alpha_0$ und $\beta_0$ des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. $\lambda$, ist die Wellenlänge des Laserlichts im Vakuum.

Die maximale Beugungseffizienz (DE = $\eta_{max}$) ergibt sich dann für $\xi$ = 0 zu:

$$DE = \tanh^2(\nu) = \tanh^2\left(\frac{\pi \cdot \Delta n \cdot d'}{\lambda \cdot \sqrt{\cos(\alpha') \cdot \cos(\alpha' - 2\psi)}}\right)$$

Die Messdaten der Beugungseffizienz, die theoretische Braggkurve und die transmittierte Intensität werden wie in Figur 2 gezeigt gegen den zentrierten Drehwinkel $\Delta\Omega = \Omega_{reconstrudion} - \Omega = \alpha'_0 - \vartheta'_0$, auch Winkeldetuning genannt, aufgetragen.

Da DE bekannt ist wird die Form der theoretischen Braggkurve nach Kogelnik nur noch durch die Dicke *d'* der Photopolymerschicht bestimmt. $\Delta$n wird über DE für gegebene Dicke *d'* so nachkorrigiert, dass Messung und Theorie von DE immer übereinstimmen. *d'* wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines $\Omega$-Scans mitrotiert, der Detektor für das abgebeugte Licht aber nur einen endlichen Winkelbereich erfassen kann, wird die Braggkurve von breiten Holgrammen (kleines *d'*) bei einem $\Omega$-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke *d'* zusätzlich herangezogen.

Figur 2 zeigt die Darstellung der Braggkurve $\eta$ nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning $\Delta\Omega$. Figur 2 zeigt die gemessene transmittierte Leistung $P_T$ (rechte *y*-Achse) als durchgezogene Linie gegen das Winkeldetuiiing $\Delta\Omega$ aufgetragen, die gemessene Beugungseffizienz $\eta$ (linke *y*-Achse) als ausgefüllte Kreise gegen das Winkeldetuning $\Delta\Omega$ aufgetragen (soweit die endliche Größe des Detektors es erlaubte) und die Anpassung der Kogelnik Theorie als gestrichelte Linie (linke y-Achse).

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis *E* ergibt sich wie folgt aus den Leistungen der zwei den Winkeln $\alpha_0$ und $\beta_0$ zugeordneten Teilstrahlen (Referenzstrahl mit $P_r$ = 0.50 mW und Signalstrahl mit $P_s$ = 0.63 mW), der Belichtungszeit t und dem Durchmesser der Irisblende (0.4 cm):

$$E\,(\mathrm{mJ/cm^2}) = \frac{2 \cdot [P_r + P_s] \cdot t\,(\mathrm{s})}{\pi \cdot 0.4^2\ \mathrm{cm}^2}$$

Die Leistungen der Teilstrahlen wurden so angepasst, dass in dem Medium bei den verwendeten Winkeln $\alpha_0$ und $\beta_0$, die gleiche Leistungsdichte erreicht wird.

<u>Herstellung der erfindungsgemäßen Methacrylate</u>

**<u>Beispiel 1.1-1.3</u>:** Allgemeine Herstellvorschrift gemäß Tabelle 1

Glycidylmethacrylat, Triphenylphosphin und Jonol (2,5-Di-tert.-butyl-4-methylphenol) werden in einem Dreihalskolben mit Rührer und Rückflußkühler vorgelegt und es wird langsam Luft durchgeleitet. Es wird auf 70 °C erwärmt.

Man gibt nun die Säure hinzu und rührt bei den angegebenen Bedingungen weiter, bis die Auswertung des $^{1}$H-NMR Spektrums zeigt, dass der Ansatz weitgehend frei von Epoxid ist (Epoxid zeigt bei Anwesenheit die charakteristischen Resonanzen bei $^{1}$H-NMR (400 MHz, CDC13): $\delta$=2,6 (dd), 2,8 (dd), 3,2 (m)).

**Beispiel 2.1-2.3:** Allgemeine Herstellvorschrift gemäß Tabelle 2

Das Produkt aus dem in der Tabelle 2 angegebenen Beispiel und Dibutylzinndilaurat werden bei 60 °C in einem Dreihalskolben mit Rührer und Rückflusskühler vorgelegt und es wird langsam Luft durchgeleitet. Innerhalb von 25 Minuten werden nun unter Exothermie das m-Methylthiophenylisocyanat zugetropft. Man rührt gemäß der angegebenen Reaktionsbedingungen und erhält das Produkt.

**Beispiel 3.1-3.3:** Allgemeine Herstellvorschrift gemäß Tabelle 3

Das Produkt aus dem in der Tabelle 3 angegebenen Beispiels und Dibutylzinndilaurat (DBTL) werden bei 60 °C in einem Dreihalskolben mit Rührer und Rückflusskühler vorgelegt und es wird langsam Luft durchgeleitet. Innerhalb von 25 Minuten wird nun unter Exothermie das Naphthylisocyanat zugetropft. Man rührt gemäß der angegebenen Reaktionsbedingungen und erhält das Produkt.

Tabelle 1

| Beispiel | Produkt | Einsatzstoffe | Reaktionsbedingungen | Beschreibung |
|---|---|---|---|---|
| 1.1 | 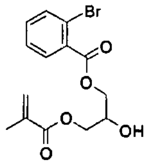 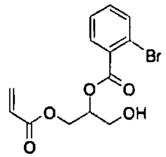<br>Mischung aus 2-hydroxy-3-[(2-methylacryloyl)oxy]propyl 2-bromobenzoate und 2-(acryloyloxyl-1-(hydroxymethyl)ethyl 2-bromobenzoate | 1.) 15,6 g Glycidylmethacrylat<br>2.) 72 mg Triphenylphosphin<br>3.) 0,4 mg Jonol<br>4.) 22,1 g 2-Brombenzoesäure | 70 °C, 42 h | Klare, farblose Flüssigkeit |
| 1.2 | 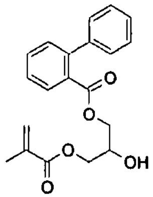 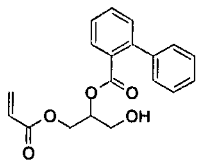<br>Mischung aus 2-hydroxy-3-[(2-methylacryloyl)oxy]propyl biphenyl-2-carboxylate und 2-(acryloyloxy)-1-(hydroxymethyl)ethyl biphenyl-2-carboxylate | 1.) 21,3 g Glycidylmethacrylat<br>2.) 98 mg Triphenylphosphin<br>3.) 15,3 mg Jonol<br>4.) 29,7 g 2-Phenylbenzoesäure | 70 °C, 52 h | Leicht gelbliche, klare, mittelviskose Flüssigkeit |
| 1.3 | 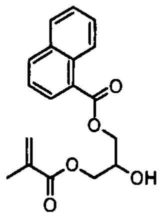 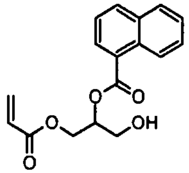<br>Mischung aus 2-hydroxy-3-[(2- methylacryloyl)oxy]propyl naphthalene-1-carboxylate und 2-(acryloyloxy)-1-(hydroxymethyl)ethyl naphthalene-1-carboxylate | 1.) 23,3 g Glycidylmethacrylat<br>2.) 107 mg Triphenylphosphin<br>3.) 15,4 mg Jonol<br>4.) 28,2 g 1-Napthoesäure | 70 °C, 44h | klare, gelbliche, viskose Flüssigkeit |

Tabelle 2:

| Beispiel | Produkt | Einsatzstoffe | Reaktionsbedingungen | Beschreibung |
|---|---|---|---|---|
| 2.1 | 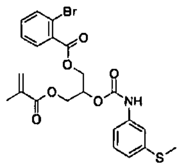 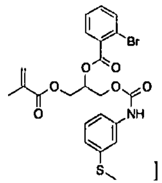 ]<br>Mischung aus 3-[(2-methylacryloyl)oxy]-2-[(3-methylthiophenylcarbamoyl)oxy]propyl 2-bromobenzoate und 2-[(2-methylacryloyl)oxy]-1-{[(3-methylthiophenylcarbamoyl)oxy]methyl}ethyl 2-bromobenzoate | 1.) 7,9 g Beispiel 1.1<br>2.) 1 mg DBTL<br>3.) 3,8 g m-Methylthiophenylisocyanat | 60 °C, 22h | Klare, cremefarbene, hochviskose Flüssigkeit |
| 2.2 | 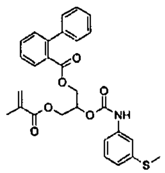 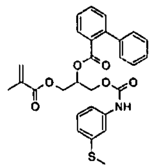<br>Mischung aus 3-[(2-methylacryloyl)oxy]-2-[(3-methylthiophenylcarbamoyl)oxy]propyl biphenyl-2-carboxylate und 2-[(2-methylacryloyl)oxy]-1-{[(3-methylthiophenylcarbamoyl)oxy]methyl} ethyl biphenyl-2-carboxylate. | 1.) 10,2 g Beispiel 1.2<br>2.) 2,0 mg DBTL<br>3.) 5,0 g m-Methylthiophenylisocyanat | 60 °C, 19h | Klare, gelbe, pastöse Masse |
| 2.3 | 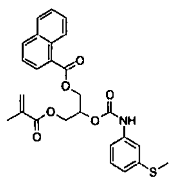 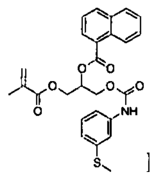 ]<br>Mischung aus 3-[(2-methylacryloyl)oxy]-2-[(3-methylthiophenylcarbamoyl)oxy]propyl naphthalene-1-carboxylate und 2-[(2-methylacryloyl)oxy]-1-{[(3-methylthiophenylcarbamoyl)oxy]methyl} ethyl naphthalene-1-carboxylate. | 1.) 9,4g Beispiel 1.3<br>2.) 1,0 mg DBTL<br>3.) 5,0g m-Methylthiophenylisocyanat | 60 °C, 22h | Hochviskose, leicht trübe Flüssigkeit |

Tabelle 3:

| Beispiel | Produkt | Einsatzstoffe | Reaktionsbedingungen | Beschreibung |
|---|---|---|---|---|
| 3.1 | 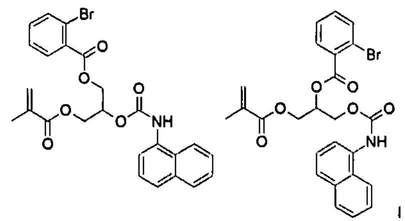 I<br>Mischung aus 3-[(2-methyacryloyl)oxy]-2-[(1-napthylcarbamoyl)oxy]propyl 2-bromobenzoate und 2-[(2-methylacryloyl)oxy]-1-{[(1-napthyl carbamoyl)oxy]methyl} ethyl 2-bromobenzoate | 1.) 7,9 g Beispiel 1.1<br>2.) 1,0 mg DBTL<br>3.) 3,9 g 1Napthylisocyanat | 60 °C, 22h | Trübe, cremefarbene hochviskose Masse |
| 3.2 | 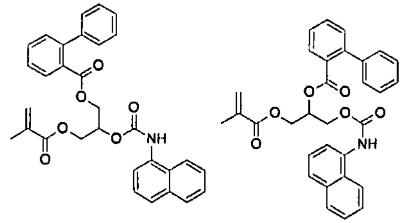<br>Mischung aus 3-[(2-methylacryloyl)oxy]-2-[(1-napthylphenylcarbamoyl)oxy]propyl biphenyl-2-carboxylate und 2-[(2-methylacryloyl)oxy]-1-{[(1-napthyl carbamoyl)oxy]methyl}ethyl biphenyl-2-carboxylate | 1.) 10,2 g Beispiel 1.2<br>2.) 2,0 mg DBTL<br>3.) 5,1g 1-Napthylisocyanat | 60 °C, 19h | Trübes, bräunliches Glas |
| 3.3 | 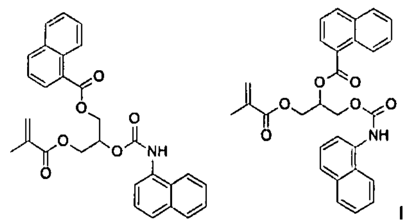 I<br>Mischung aus 3-[(2-methylacryloyl)oxy]-2-[(1-napthylcarbamoyl)oxy]propyl naphthalene-1-carboxylate und 2-[(2-methylacryloyl)oxy]-1-{[(1-napthylcarbamoyl)oxy]methyl}ethyl naphthalene-1-carboxylate | 1.) 5,9 g Beispiel 1.3<br>2.) 1,0 mg DBTL<br>3.) 3,2 g 1-Napthylisocyanat | 60 °C, 21,5 h | Trübes, bräunliches Glas |

Herstellung des Polyols

**Beispiel 4.0**

In einem 1 L Kolben werden 18 g Zinnoktoat, 374.8 g ε-Caprolacton und 374.8 g eines difunktionel-len Polytetrahydrofuranpolyetherpolyols (Equivalentgewicht 500 g/Mol OH, z.B. Terathane® 1000 ein Produkt der BASF SE, Ludwigshafen DE) vorgelegt und auf 120 °C aufgeheizt und so lange auf dieser Temperatur gehalten, bis der Festkörpergehalt bei 99.5 Gew.-% oder darüber lag (Anteil der nicht-flüchtigen Bestandteile bestimmt durch eine Stunde Ofenlagerung von einem Gramm des Produktes in einem unlackierten Ofendeckel bei 125 °C, berechnet nach den gravimetrischen Ergebnissen : Auswaage [g] * 100 / Einwaage [g] = Gew.-% Festkörper). Anschließend wurde abgekühlt und das Produkt als wachsartiger Feststoff erhalten.

Herstellung der holografischen Medien

**Beispiele 5.1-5.6**

5.927 g der wie oben beschrieben hergestellten Polyol-Komponente (Beispiel 4.0) wurden mit 2.50 g des Produkts aus Beispiels 2.1, 0.10 g CGI-909 (Tetrabutylammonium-tris(3-chlor-4-methylphenyl)(hexyl)borat, [1147315-11-4]), ein von der CIBA Inc., Basel, Schweiz, hergestelltes Versuchsprodukt, 0,015g Glasperlen der Größe 20 μm (Whitehouse Scientific Ltd, Waverton, Chester, CH3 7PB, United Kingdom), 0.010 g Neu Methylenblau bei 60 °C und 0,35 g N-Ethylpyrilidon gemischt so dass eine klare Lösung erhalten wurde. Anschließend wurde auf 30 °C abgekühlt, 1.098 g Desmodur® XP 2410 (Versuchsprodukt der Bayer MaterialScience AG, Leverkusen, Deutschland, Hexandiisocyanat-basiertes Polyisocyanat, Anteil an Iminooxadiazindion mindestens 30 %, NCO-Gehalt: 23.5 %) zugegeben und erneut gemischt. Schließlich wurden 0.006 g Fomrez UL 28 (U-rethanisierungskatalysator, Handelsprodukt der Momentive Performance Chemicals, Wilton, CT, USA) zugegeben und erneut kurz gemischt (mittels Speedmixer). Die erhaltene, flüssige Masse wurde dann auf eine Glasplatte gegeben und dort mit einer zweiten Glasplatte abgedeckt. Die Härtung der PU-Formulierung erfolgt unter 15 kg Gewichten über mehrere Stunden (gewöhnlich über Nacht). Es wird ein formstabiler Glassandwich (Coupon) erhalten. Da unterschiedliche Formulierungen mit unterschiedlicher Ausgangsviskosität und unterschiedlicher Härtungsgeschwindigkeit der Matrix zu nicht immer gleichen Schichtdicken *d'* der Photopolymerschicht fuhren, wird *d'* anhand der Charakteristika der geschriebenen Hologramme für jede Probe separat ermittelt.

Die Medien 5.2-5.6 wurden in analoger Art und Weise aus den in Tabelle 2 und 3 aufgeführten Beispielen hergestellt.

Tabelle 4: Ergebnisse der holografischen Ausprüfung der erfindungsgemäßen Methacrylate als Schreibmonomer in den erfindungsgemäßen Photopolymeren.

| $R^2$\$R^1$ | 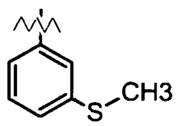 | 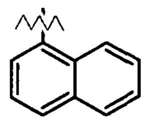 |
|---|---|---|
| 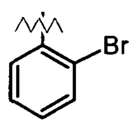 | Beispiel 5.1<br>Methacrylat aus Beispiel 2.1<br>Dn=0,0026<br>Belichtungszeit 4 s<br>Energiedosis 18,22 mJ/$cm^2$<br>Schichtdicke 26,4 μm | Beispiel 5.4<br>Methacrylat aus Beispiel 3.1<br>Dn=0,0049<br>Belichtungszeit 1 s<br>Energiedosis 4,56 mJ/$cm^2$<br>Schichtdicke 30,0 μm |
| 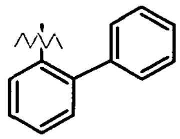 | Beispiel 5.2<br>Methacrylat aus Beispiel 2.2<br>Dn=0,0063<br>Belichtungszeit 1 s<br>Energiedosis 4,56 mJ/$cm^2$<br>Schichtdicke 20,5 μm | Beispiel 5.5<br>Methacrylat aus Beispiel 3.2<br>Dn=0,0099<br>Belichtungszeit 1 s<br>Energiedosis 4,56 mJ/$cm^2$<br>Schichtdicke 16,0 μm |

(fortgesetzt)

| $R^2$\$R^1$ | 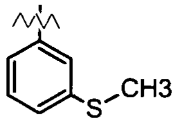 | 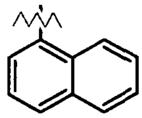 |
|---|---|---|
| 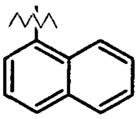 | Beispiel 5.3<br>Methacrylat aus Beispiel 2.3<br>Dn=0,0080<br>Belichtungszeit 1 s<br>Energiedosis 4,46 mJ/cm$^2$<br>Schichtdicke 17,0 µm | Beispiel 5.6<br>Methacrylat aus Beispiel 3.3<br>Dn=0,0094<br>Belichtungszeit 1 s<br>Energiedosis 4,56 mJ/cm$^2$<br>Schichtdicke 11,5 µm |

**<u>Beispiel 6.0 -</u> Herstellung eines flourierten Weichmachers** [Bis(2,2,3,3,4,4,5,5,6,6,7,7-dodecafluorheptyl)-(2,2,4-trimethylhexan-1,6-diyl)biscarbamat]

In einem Dreihals-Rundkolben mit Rückflußkühler und Rührer wurden 0.02 g Desmorapid Z (Dibutylzinndilaurat) und 3,60 g 2, 4, 4-Trimethylhexane-1,6-diisocyanat (TMDI) vorgelegt und auf 70 °C erwärmt. Anschließend wurden 11,39 g 1H,1H-7H-Perfluoroheptan-1-ol zugetropft und die Mischung weiter auf 70 °C gehalten, bis der Isocyanatgehalt unter 0.1 % gesunken war. Danach wurde abgekühlt. Das Produkt wurde als farbloses Öl erhalten.

**<u>Beispiel 7.1</u>- Verwendung eines fluorierten Weichmachers**

Analog der Durchführung in Beispiel 5.1-5.6 werden 3,792 g des Polyols aus Beispiel 4.0, 2,500 g des Beispiel 3.3,2,500 g des fluorierten Weichmachers aus Beispiel 6.0, 0.1 g CGI-909 (Tetrabutylammonium-tris(3-chlor-4-methylphenyl)(hexyl)borat), 0,015g Glasperlen der Größe 20 µm, 0.01 g Neu Methylenblau bei 60 °C und 0,345 g N-Ethylpyrilidon gemischt so dass eine klare Lösung erhalten wurde. Anschließend wurde auf 30 °C abgekühlt, 0,702 g Desmodur® XP 2410 zugegeben und erneut gemischt. Schließlich wurden 0.006 g Fomrez UL 28 zugegeben und erneut kurz gemischt (mittels Speedmixer). Man erhält die folgende holografischen Performance: Dn=0,0244 / 4 s Belichtungsdauer/ Energiedosis 18,1 mJ/cm$^2$ / 12,0 µm berechnete Schichtdicke.

**<u>Beispiel 7.2-</u> Verwendung eines fluorierten Weichmachers**

Analog der Durchführung in Beispiel 7.1 werden 3,370g des Polyols aus Beispiel 4.0, 4,000 g des Beispiel 3.3, 1,500 g des fluorierten Weichmachers aus Beispiel 6.0 und 0,624 g Desmodur® XP 2410 verwendet. Die anderen Komponenten werden in gleicher Menge verwendet. Man erhält die folgende holografischen Performance: Dn=0,0265 / 2 s Belichtungsdauer/ Energiedosis 9,11 mJ/cm$^2$ / 18,0 µm berechnete Schichtdicke.

**<u>Beispiel 8.1 -</u> Herstellung eines nicht erfindungsgemäßen Schreibmonomer - Phosphorothioyltris(oxybenzol-4,1-diylcarbamoyloxyethan-2,1-diyl)trisacrylat**

In einem 500 mL Rundkolben wurden 0.1 g 2,6-Di-tert.-butyl-4-methylphenol, 0.05 g Dibutylzinndilaurat (Desmorapid Z, Bayer MaterialScience AG, Leverkusen, Deutschland) sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

**<u>Beispiel 8.2-</u> Verwendung eines weiteren Schreibmonomers**

Analog der Durchführung in Beispiel 7.1 werden 5,901 g des Polyols aus Beispiel 4.0, 1,500 g des erfindungsgemäßen Schreibmonomers aus Beispiel 3.2, 1,000 g des nicht erfindungsgemäßen Schreibmonomers aus Beispiel 8.1 und 1,093 g Desmodur® XP 2410 verwendet. Die anderen Komponenten werden in gleicher Menge verwendet. Man erhält die folgende holografischen Performance: Dn=0,0061 / 4 s Belichtungsdauer / Energiedosis 18,22 mJ/cm$^2$ / berechnete Schichtdicke 25,0 µm.

**Beispiel 8.3- Verwendung eines weiteren Weichmachers**

Analog der Durchführung in Beispiel 7.1 werden 4,636 g des Polyols aus Beispiel 4.0, 2,500 g des erfindungsgemäßen Schreibmonomers aus Beispiel 3.2, 1,500 g des Weichmachers aus Beispiel 6.0 und 0,859 g Desmodur® XP 2410 verwendet. Die anderen Komponenten werden in gleicher Menge verwendet. Man erhält die folgende holografischen Performance: Dn=0,0060 / 4 s Belichtungsdauer/ Energiedosis 18,22 mJ/$cm^2$ / berechnete Schichtdicke 15,0 $\mu$m.

**Beispiel 8.4 - Verwendung eines weiteren Schreibmonomers und eines fluorierten Weichmachers**

Analog der Durchführung in Beispiel 7.1 werden 4,636 g des Polyols aus Beispiel 4.0, 1,500 g des erfindungsgemäßen Schreibmonomers aus Beispiel 3.2, 1,000 g des nicht erfindungsgemäßen Schreibmonomers aus Beispiel 8.1, 1,500 g des Weichmachers aus Beispiel 6.0 und 0,859 g Desmodur® XP 2410 verwendet. Die anderen Komponenten werden in gleicher Menge verwendet. Man erhält die folgende holografischen Performance: Dn=0,0026 / 8 s Belichtungsdauer /Energiedosis 36,45 mJ/$cm^2$/ berechnete Schichtdicke 17,0 $\mu$m.

Wie Tabelle 4 sowie Beispiele 7.1, 7.2 und 8.2-8.4 zeigen, haben die erfindungsgemäßen holografischen Medien eine gute holografische Performance. Die Indexmodulation beträgt zwischen 0,0026 und 0,0265. Zudem ist die Herstellung der erfindungsgemäßen Methacrylate (Beispiele 1.1 - 3.3) einfach durchführbar, insbesondere ist kein Destillationsschritt notwendig.

## Patentansprüche

1. Methacrylat der allgemeinen Formeln (I) oder (II) sowie Mischungen daraus

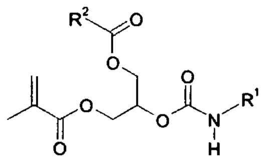

(I)

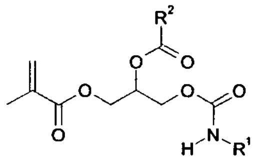

(II) ,

**dadurch gekennzeichnet, dass** $R^1$ und $R^2$ unabhängig voneinander substituierte Phenyl-, substituierte und / oder unsubstituierte Naphthylreste sind.

2. Methacrylat nach Anspruch 1, **dadurch gekennzeichnet, dass** $R^1$ und / oder $R^2$ 6-24 C-Atome, 0-5 S-Atome und 0-5 Halogenatome umfassen.

3. Methacrylat nach einem der Ansprüche 1 oder 2, **dadurch gekennzeichnet, dass** $R^1$ und /oder $R^2$ mit Thioethergruppen, Phenylgruppen und / oder Halogenatome substituiert sind.

4. Methacrylat nach einem der Ansprüche 1 bis 3, **dadurch gekennzeichnet, dass** $R^1$ und / oder $R^2$ Napthyl, 3-Methylthiophenyl, 2-, 3-, 4-Biphenyl, 2-Bromphenyl sind.

5. Verfahren zur Herstellung eines Methacrylats gemäß Anspruch 1, **dadurch gekennzeichnet, dass** eine aromatische Säure $R^2$-COOH mit Glycidylmethacrylat zur Reaktion gebracht und das Produkt anschließend mit einem aromatischen Isocyanat $R^1$-NCO umgesetzt wird

6. Photopolymer-Formulierung umfassend Matrixpolymere, Schreibmonomere und Photoinitiatoren, **dadurch gekennzeichnet, dass** die Schreibmonomere ein Methacrylat nach einem der Ansprüche 1 bis 4 beinhalten.

7. Photopolymer-Formulierung nach Anspruch 6, **dadurch gekennzeichnet, dass** die Matrixpolymere Polyurethane sind.

8. Photopolymer-Formulierung nach einem der Ansprüche 6 oder 7, **dadurch gekennzeichnet, dass** die Photoinitiatoren einen anionischen, kationischen oder neutralen Farbstoff und einen Coinitiator umfassen.

9. Photopolymer-Formulierung nach einem der Ansprüche 6 bis 8, **dadurch gekennzeichnet, dass** sie zusätzlich

Urethane als Weichmacher enthält, wobei die Urethane insbesondere mit wenigstens einem Fluoratom substituiert sein können.

**10.** Photopolymer-Formulierung nach Anspruch 9, **dadurch gekennzeichnet, dass** die Urethane die allgemeine Formel (III)

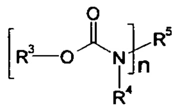

(III)

haben, in der n≥1 und n≤8 ist und $R^3$, $R^4$, $R^5$ Wasserstoff und / oder unabhängig voneinander lineare, verzweigte, cyclische oder heterocyclische unsubstituierte oder gegebenenfalls auch mit Heteroatomen substituierte organische Reste sind, wobei bevorzugt mindestens einer der Reste $R^3$, $R^4$, $R^5$ mit wenigstens einem Fluoratom substituiert ist und besonders bevorzugt $R^3$ ein organischer Rest mit mindestens einem Fluoratom ist.

**11.** Photopolymer-Formulierung nach einem der Ansprüche 5 bis 10, **dadurch gekennzeichnet, dass** die Schreibmonomere zusätzlich ein multifunktionales Schreibmonomer umfassen, wobei es sich insbesondere um ein multifunktionelles Acrylat handeln kann.

**12.** Photopolymer-Formulierung nach Anspruch 11, **dadurch gekennzeichnet, dass** das multifunktionelle Acrylat die allgemeine Formel (IV)

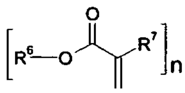

(IV)

hat, in der n≥2 und n≤4 ist und $R^6$, $R^7$ Wasserstoff und / oder unabhängig voneinander lineare, verzweigte, cyclische oder heterocyclische unsubstituierte oder gegebenenfalls auch mit Heteroatomen substituierte organische Reste sind.

**13.** Verwendung einer Photopolymer-Formulierung nach einem der Ansprüche 6 bis 12 zur Herstellung holographischer Medien, insbesondere zur Herstellung von In-Line Hologrammen, Off-Axis Hologrammen, Full-Aperture Transfer Hologrammen, Weißlicht-Transmissionshologrammen, Denisyukhologrammen, Off-Axis Reflektionshologrammen, Edge-Lit Hologrammen sowie holographischen Stereogrammen.

## Claims

**1.** Methacrylate of the general formulae (I) or (II) and mixtures thereof

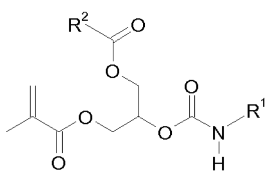

(I)

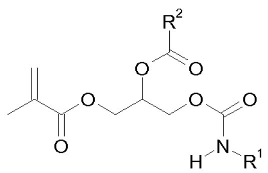

(II)

,

**characterized in that** $R^1$ and $R^2$, independently of one another, are substituted phenyl radicals, substituted and/or unsubstituted naphthyl radicals.

**2.** Methacrylate according to Claim 1, **characterized in that** $R^1$ and/or $R^2$ comprise 6-24 C atoms, 0-5 S atoms and 0-5 halogen atoms.

**3.** Methacrylate according to either of Claims 1 and 2, **characterized in that** $R^1$ and/or $R^2$ are substituted by thioether

groups, phenyl groups and/or halogen atoms.

4. Methacrylate according to any of Claims 1 to 3, **characterized in that** $R^1$ and/or $R^2$ are naphthyl, 3-methylthiophenyl, 2-, 3- or 4-biphenyl, 2-bromophenyl.

5. Process for the preparation of a methacrylate according to Claim 1, **characterized in that** an aromatic acid $R^2$-COOH is reacted with glycidyl methacrylate and the product is then reacted with an aromatic isocyanate $R^1$-NCO.

6. Photopolymer formulation comprising matrix polymers, writing monomers and photoinitiators, **characterized in that** the writing monomers comprise a methacrylate according to any of Claims 1 to 4.

7. Photopolymer formulation according to Claim 6, **characterized in that** the matrix polymers are polyurethanes.

8. Photopolymer formulation according to either of Claims 6 and 7, **characterized in that** the photoinitiators comprise an anionic, cationic or neutral dye and a coinitiator.

9. Photopolymer formulation according to any of Claims 6 to 8, **characterized in that** they additionally contain urethanes as plasticizers, it being possible for the urethanes to be substituted in particular by at least one fluorine atom.

10. Photopolymer formulation according to Claim 9, **characterized in that** the urethanes have the general formula (III)

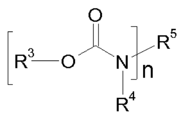

(III)

in which n≥1 and n≤8 and $R^3$, $R^4$, $R^5$ are hydrogen and/or, independently of one another, linear, branched, cyclic or heterocyclic organic radicals which are unsubstituted or optionally also substituted by heteroatoms, preferably at least one of the radicals $R^3$, $R^4$, $R^5$ being substituted by at least one fluorine atom and particularly preferably $R^3$ being an organic radical having at least one fluorine atom.

11. Photopolymer formulation according to any of Claims 5 to 10, **characterized in that** the writing monomers additionally comprise a polyfunctional writing monomer, it being possible for this to be in particular a polyfunctional acrylate.

12. Photopolymer formulation according to Claim 11, **characterized in that** the polyfunctional acrylate has the general formula (IV)

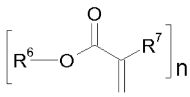

(IV)

in which n≥2 and n≤4 and $R^6$, $R^7$ are hydrogen and/or, independently of one another, linear, branched, cyclic or heterocyclic organic radicals which are unsubstituted or optionally also substituted by heteroatoms.

13. Use of a photopolymer formulation according to any of Claims 6 to 12 for the production of holographic media, in particular for the production of in-line holograms, off-axis holograms, full-aperture transfer holograms, white light transmission holograms, Denisyuk holograms, off-axis reflection holograms, edge-lit holograms and holographic stereograms.

**Revendications**

1. Méthacrylate de formule générale (I) ou (II) et leurs mélanges

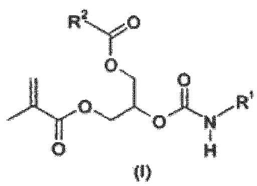
(I)

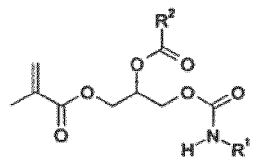
(II)

,

**caractérisé en ce que** $R^1$ et $R^2$ sont indépendamment l'un de l'autre des radicaux phényle substitués, naphtyle substitués et/ou non substitués.

2. Méthacrylate selon la revendication 1, **caractérisé en ce que** $R^1$ et/ou $R^2$ comprennent 6 à 24 atomes C, 0 à 5 atomes S et 0 à 5 atomes d'halogène.

3. Méthacrylate selon l'une quelconque des revendications 1 ou 2, **caractérisé en ce que** $R^1$ et/ou $R^2$ sont substitués avec des groupes thioéther, des groupes phényle et/ou des atomes d'halogène.

4. Méthacrylate selon l'une quelconque des revendications 1 à 3, **caractérisé en ce que** $R^1$ et/ou $R^2$ représentent naphtyle, 3-méthylthiophényle, 2-, 3-, 4-biphényle, 2-bromophényle.

5. Procédé de fabrication d'un méthacrylate selon la revendication 1, **caractérisé en ce qu'**un acide aromatique $R^2$-COOH est mis en réaction avec du méthacrylate de glycidyle, puis le produit est mis en réaction avec un isocyanate aromatique $R^1$-NCO.

6. Formulation de photopolymère comprenant des polymères de matrice, des monomères d'écriture et des photoinitiateurs, **caractérisée en ce que** les monomères d'écriture contiennent un méthacrylate selon l'une quelconque des revendications 1 à 4.

7. Formulation de photopolymère selon la revendication 6, **caractérisée en ce que** les polymères de matrice sont des polyuréthanes.

8. Formulation de photopolymère selon l'une quelconque des revendications 6 ou 7, **caractérisée en ce que** les photoinitiateurs comprennent un colorant anionique, cationique ou neutre et un co-initiateur.

9. Formulation de photopolymère selon l'une quelconque des revendications 6 à 8, **caractérisée en ce qu'**elle contient également des uréthanes en tant que plastifiants, les uréthanes pouvant notamment être substitués avec au moins un atome de fluor.

10. Formulation de photopolymère selon la revendication 9, **caractérisée en ce que** les uréthanes ont la formule générale (III)

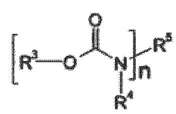
(III)

dans laquelle $n \geq 1$ et $n \leq 8$, et $R^3$, $R^4$, $R^5$ représentent l'hydrogène et/ou, indépendamment les uns des autres, des radicaux organiques linéaires, ramifiés, cycliques ou hétérocycliques non substitués ou éventuellement également substitués avec des hétéroatomes, au moins un des radicaux $R^3$, $R^4$, $R^5$ étant de préférence substitué avec au moins un atome de fluor et $R^3$ étant de manière particulièrement préférée un radical organique comprenant au moins un atome de fluor.

11. Formulation de photopolymère selon l'une quelconque des revendications 5 à 10, **caractérisée en ce que** les monomères d'écriture comprennent également un monomère d'écriture multifonctionnel, celui-ci pouvant notamment être un acrylate multifonctionnel.

12. Formulation de photopolymère selon la revendication 11, **caractérisée en ce que** l'acrylate multifonctionnel a la

formule générale (IV)

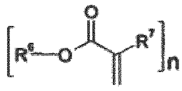 (IV)

dans laquelle n ≥ 2 et n ≤ 4, et $R^6$, $R^7$ représentent l'hydrogène et/ou, indépendamment les uns des autres, des radicaux organiques linéaires, ramifiés, cycliques ou hétérocycliques non substitués ou éventuellement également substitués avec des hétéroatomes.

**13.** Utilisation d'une formulation de photopolymère selon l'une quelconque des revendications 6 à 12 pour la fabrication de matériaux holographiques, notamment pour la fabrication d'hologrammes directs, d'hologrammes indirects, d'hologrammes de transfert à pleine ouverture, d'hologrammes en lumière blanche, d'hologrammes Denisyuk, d'hologrammes indirects par réflexion, d'hologrammes lumineux et de stéréogrammes holographiques.

**Figur 1**

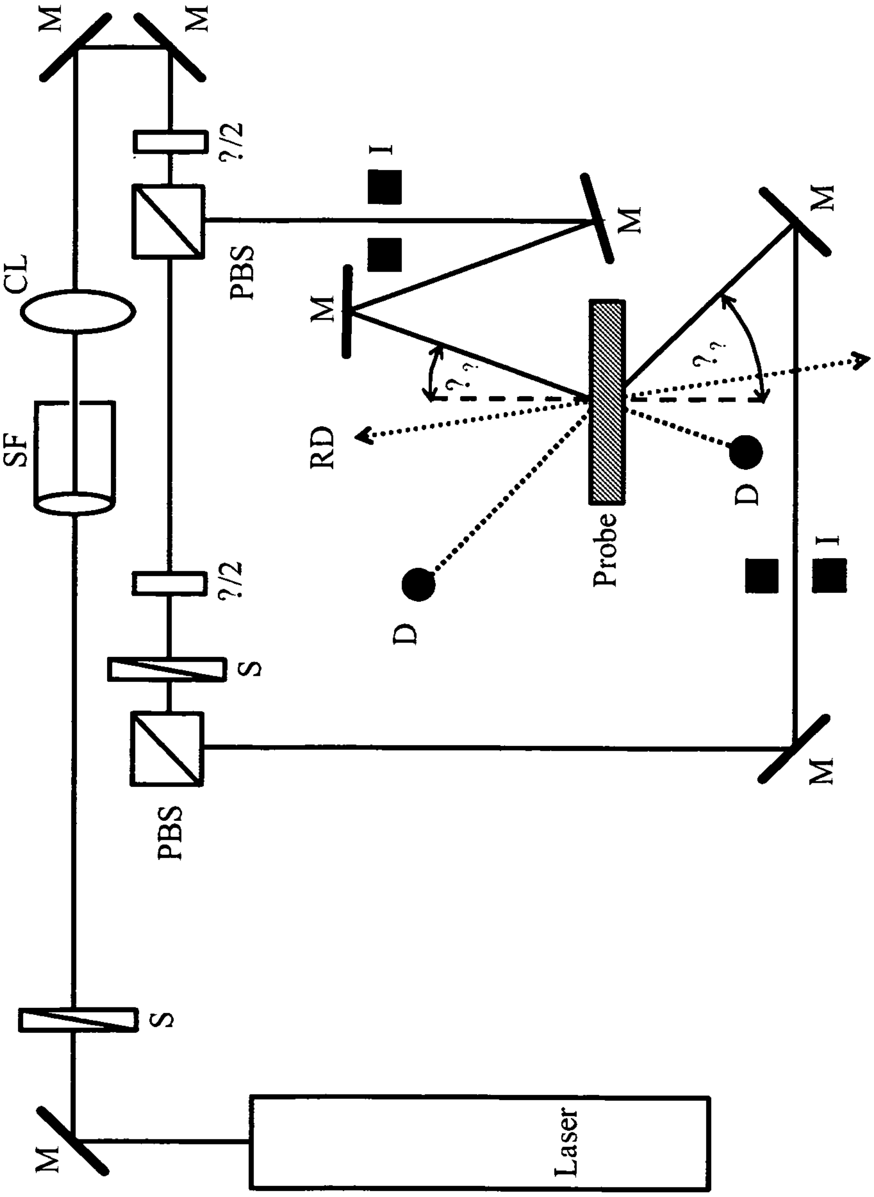

Figur 2:

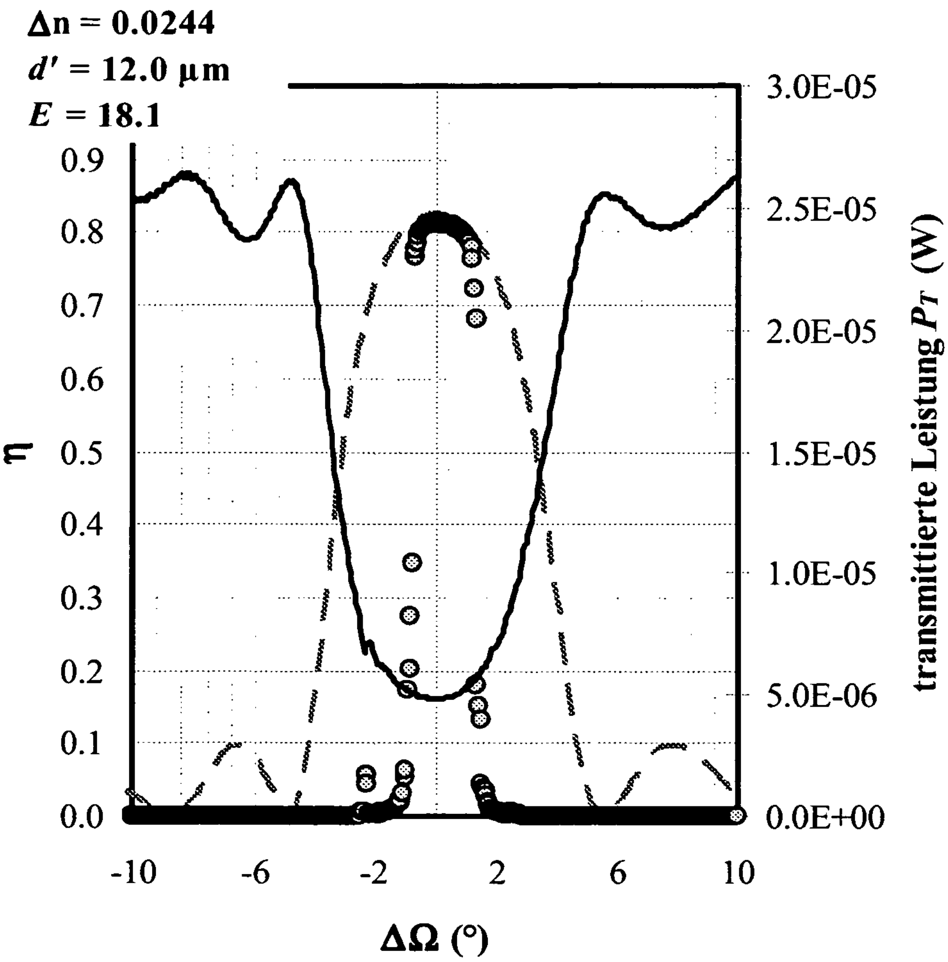

Δn = 0.0244
d' = 12.0 µm
E = 18.1
η
0.9
0.8
0.7
0.6
0.5
0.4
0.3
0.2
0.1
0.0
3.0E-05
2.5E-05
2.0E-05
1.5E-05
1.0E-05
5.0E-06
0.0E+00
transmittierte Leistung $P_T$ (W)
-10
-6
-2
2
6
10
ΔΩ (°)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125199 A1
- EP 0223587 A


### In der Beschreibung aufgeführte Nicht-Patentliteratur


- **CUNNINGHAM et al.** *RadTech'98 North America UV/EB Conference Proceedings,* 19. April 1998
- **KUTAL et al.** *Macromolecules,* 1991, vol. 24, 6872
- **YAMAGUCHI et al.** *Macromolecules,* 2000, vol. 33, 1152
- **NECKERS et al.** *Macromolecules,* 2000, vol. 33, 7761
- **LI et al.** *Polymeric Materials Science and Engineering,* 2001, vol. 84, 139
- **DEKTAR et al.** *J. Org. Chem,* 1990, vol. 55, 639
- *J. Org. Chem.,* 1991, vol. 56, 1838
- **CRIVELLO et al.** *Macromolecules,* 2000, vol. 33, 825
- **GU et al.** *Am. Chem. Soc. Polymer Preprints,* 2000, vol. 41 (2), 1266
- **HUA et al.** *Macromolecules,* 2001, vol. 34, 2488-2494
- Chemistry & Tech-nology of UV & EB Formulations For Coatings, Inks & Paints. SITA Tech-nology, 1991, vol. 3, 61-328
- **HOUBEN-WEYL.** Methoden der organischen Chemie. Georg Thieme Verlag, 1961, vol. XIV/1, 433ff
- **WILLIE W. ; HONG, CHI-SHAIN ; YARIV, AMNON.** Holographie interference lithography for integrated optics. *IEEE Transactions on Electron Devices,* 1978, vol. ED-25 (10), ISSN 0018-9383, 1193-1200
- **H. KOGELNIK.** *The Bell System Technical Journal,* November 1969, vol. 48 (9), 2909, , 2947